# EUROPEAN PATENT APPLICATION

(11) **EP 3 572 097 A1**
(43) Date of publication of application: **27.11.2019**
(21) Application number: 19175563.6
(22) Date of filing: 21.05.2019
(51) Int. Cl.: A61L 2/20, A61L 2/18, A61L 2/24

(54) **WASHING APPLIANCE ADAPTED FOR APPLICATION IN PHARMACEUTICAL PRODUCTION AND/OR PRECLINICAL PHARMACEUTICAL RESEARCH CENTRES, FOR WASHING PARTS AND COMPONENTS FOR PHARMACEUTICAL PRODUCTION, AND METHOD OF USE OF THE APPLIANCE**

(30) Priority: 23.05.2018 IT 201800005614
(71) Applicant: IWT S.r.L., 21020 Casale Litta (IT)
(72) Inventor: BORGHI, Matteo, 21020 Casale Litta (VA) (IT); PAGANI, Marco, 21020 Casale Litta (VA) (IT)
(74) Representative: Borsano, Corrado

(57) **Abstract**

A washing appliance is described, which is adapted for application in pharmaceutical production and/or preclinical pharmaceutical research centres, and adapted for the execution of washing/rinsing steps on parts and components for pharmaceutical production, comprising a hydraulic circuit for the circulation of washing/rinsing liquid and a recirculation pump (8) in the hydraulic circuit, characterized in that it comprises:
- a wash chamber (1) adapted to contain said parts and components for pharmaceutical production to be washed, said chamber comprising one or two hermetically closed access doors (20);
- one or more washing systems, internal to the wash chamber and connected to said hydraulic circuit, and adapted to supply wash liquid into the chamber;
- an accumulation basin (2) at the bottom of the wash chamber, for accumulating the washing/rinsing liquid, connected to said recirculation pump (8);
- a heat exchanger (10) for heating or maintaining the temperature of the washing/rinsing liquid received through said recirculation pump (8) and supplied to said one or more washing systems;
- a generation system (15) adapted to generate gaseous ozone;
- mixing means adapted to mix said gaseous ozone with the washing/rinsing liquid and arranged in said hydraulic circuit;
- a control system (18) adapted to control said washing/rinsing steps of the appliance, and adapted to control said gaseous ozone generation system (15) for mixing said gaseous ozone during any one of said washing/rinsing steps of the appliance.

## Description

### Field of application of the invention

The present invention relates to a method, and an appliance for implementing the method, for washing parts and components for pharmaceutical production.

### State of the art

In the field of pharmaceutical production, it is known that washing systems need to be used in order to remove product or contaminants from drug production parts and various components such as: bins, press punches, drug injection pumps, tubing and valve parts, etc. It is also known that washing procedures must be carried out in order to remove pharmaceutical product or contaminants from bins used for transforming and storing pharmaceutical products. Such procedures must be carried out both during normal single-product production and, in particular, when using the same systems/components in multi-product lines. In this latter case, the washing procedure must ensure, prior to producing the next product, the elimination of any product residues and contaminants from the parts subjected to the washing cycle and also from those parts of the washing machine which have come in contact with the liquid used during the various cycle steps.

The washing procedures known in the art are of two types:
1) Manual: by executing a special procedure using water jets and detergent, the operator manually removes dirt from parts/components/bins; the same operator then rinses such parts by using the same technology.
2) Automatic: part and/or bin washing machines are used; in this case, the parts/components/bins are housed inside a suitable wash chamber which, by means of dedicated washing and rinsing systems, removes the dirt according to an accurate and reliable sequence.

The automatic washing machines available on the market typically operate in accordance with a procedure that, depending on the nature of the product to be removed from the parts to be washed, includes the following steps:
- The components to be washed are positioned inside the wash chamber in positions that are usually determined in such a way as to ensure good load coverage by the washing liquid in use.
- Once the machine, which is typically composed of a wash chamber, an accumulation basin and a sanitary recirculation circuit (centrifugal pump, self-draining tubing and sanitary-quality components), has been loaded with parts to be washed, the accumulation basin is filled with water directly from the customer's water circuit (loop), wherein water quality and temperature depend on the different cycle steps and on the equipment available along the customer's lines.
- Once water has been supplied, the centrifugal pump starts recirculating the washing liquid onto the load by sucking it from the accumulation pump, through the use of simple standard washing systems (rotary rods or sprayer nozzles) or load-specific washing systems. During this first recirculation sequence, it is usually possible to increase the water temperature, if necessary, by means of a steam or electric heat-exchanger for indirect heat exchange.
- During this first sequence it is also possible to dose chemicals (detergents) for removing specific dirt and/or abating the contaminant charge.
- When the necessary temperature and dosage values have been reached, pressure is increased to the value set in the recipe and a countdown begins for this cycle step.
- During this step, the machine may alternately feed different final washing circuits, so that the components can be washed both internally and externally, or anyway wherever necessary.
- When this time has elapsed, the pump stops and, through a drain valve, the used bath (often hot and containing detergent) is drained towards a suitable pit available at the customer's site.
- When this step is complete, it can be repeated "n" times with different types of water, temperature and detergents; those steps which use detergents are usually identified as washing steps.
- The washing steps are followed by rinsing steps, which are identical as far as the sequence is concerned, but do not use any detergent, so as to remove all residual traces of detergent from the components and from the machine itself.
- Due to the concentrations used and water purity, it is often necessary to repeat these rinsing steps "n" times until the water conductivity value returns to a value close to that of the supplied water; all this is usually controlled by the machine itself through a suitable conductivity probe. If conductivity does not meet the requirements, this step will be repeated again.
- Lastly, a final step may be necessary/required with ultra-pure water directly injected into the washing terminals (i.e. without first delivering it into the accumulation basin and without using the recirculation pump) to ensure that the load is rinsed with water not contaminated with any residues in the machine.

This type of procedure is usually employed for part washing machines suitable for washing components and equipped with circuits for external and internal washing through a dedicated basket connected to the washing cycle by means of a "quick-lock" device, and also for large machines used for washing pharmaceutical bins, called bin washing machines. Another method which is mostly used for washing bins makes use of a direct open circuit: in this case, the water taken in from the customer's loop is directly pressurized, without using an accumulation basin, and sprayed onto the load to be washed, possibly with in-line detergent injection and in-line heating. After hitting the load, the water falls and is directly conveyed into the drain pit.

It is often required that the parts, after having been washed and before returning to the production departments, be decontaminated at high temperatures (approx. 120-130 °C); this process usually occurs in other dedicated machines called autoclaves.

Both types of automatic washing processes (recirculation and open circuit) have some advantages and disadvantages.

Recirculation washing: this system offers more flexibility and allows for more appropriate washing rates and pressures. Moreover, it allows washing many components together, since many washing nozzles/systems can be simultaneously present aboard the machine.

Since chemicals (detergents) need to be used in order to remove/decontaminate the components, it is necessary to use hot water (when injected into cold water, detergents produce foam, resulting in pump cavitation and reduced mechanical washing performance), and therefore energy.

Recirculation inevitably results in pockets of residue accumulation between one step and another: notwithstanding the self-draining design of the machines and the use of specific components, it is currently impossible to ensure the complete absence of even minimal accumulation areas. According to design rules, in fact, some minimum accumulation values are considered acceptable. It is therefore mandatory to carry out costly repetitions of the rinsing steps with water that is increasingly bacteriologically pure, which is however contaminated with previous residues.

For this reason, the final rinsing must often be carried out with ultra-pure water; however, this step poses a number of problems.

First of all, the presence of this type of water in the customer's systems, considering the complexity and production cost thereof.

Secondly, where this ultra-pure water is available it is still necessary to take into account that, in order to directly supply washing circuits designed for high flow rates, a line providing significant flow rates must be available. This is very difficult to obtain, because these are ultra-filtered lines with very high production costs.

Thirdly, even when flow rates are sufficient to ensure load coverage, there is still a problem as regards the proper rinsing of the machine (with its accumulation tank and associated piping).

Therefore, in order to avoid any contamination between one cycle and the next, an unloaded self-cleaning cycle is sometimes carried out, which inevitably results in downtime and water and energy consumption.

As an alternative, clean steam (when available) is injected into the machine, which results in problems related to consumption and, most importantly, safety, since a sealed chamber, not just a piping, needs to be treated.

Open circuit washing: this is a less flexible solution, because of its limited flow rates and coverage: this type of washing is mostly used for washing specific components (bins), where a single washing point is used along with low flow rates.

This solution requires that good lines are available, with appropriate water flow rates for feeding the circuits.

Basically, the washing/rinsing steps do not last very long, due to the significant instantaneous consumption of water.

In this case, contamination between two different steps can be avoided as concerns the load to be washed. If a wash chamber is present, however, the latter is not (unlike recirculation washing) fully hit by the washing/rinsing jets, and therefore the chamber itself becomes a receptacle of contaminants, since it has no effective self-cleaning system.

Unloaded self-cleaning cycles may be carried out, which are however resource, power and time intensive, and anyway coverage is still poor because the circuits are dedicated to covering the components, not the chamber.

### Summary of the invention

It is therefore one object of the present invention to provide a washing appliance adapted for application in pharmaceutical production and/or preclinical pharmaceutical research centres, for washing parts and components for pharmaceutical production, and a method of use of the appliance, which are aimed at overcoming all of the above-mentioned drawbacks.

The present invention relates to a washing appliance adapted for application in pharmaceutical production and/or preclinical pharmaceutical research centres, and adapted for the execution of washing/rinsing steps on parts and components for pharmaceutical production, comprising a hydraulic circuit for the circulation of washing/rinsing liquid and a recirculation pump in the hydraulic circuit, characterized in that it comprises:
- a wash chamber adapted to contain said parts and components for pharmaceutical production to be washed, said chamber comprising one or two hermetically closed access doors;
- one or more washing systems, internal to the wash chamber and connected to said hydraulic circuit, and adapted to supply washing liquid into the chamber;
- an accumulation basin at the bottom of the wash chamber, for accumulating the washing/rinsing liquid, connected to said recirculation pump;
- a heat exchanger for heating or maintaining the temperature of the washing/rinsing liquid received through said recirculation pump and supplied to said one or more washing systems;
- a generation system adapted to generate gaseous ozone;
- mixing means adapted to mix said gaseous ozone with the washing/rinsing liquid and arranged in said hydraulic circuit;
- a control system adapted to control said washing/rinsing steps of the appliance and adapted to control said gaseous ozone generation system for mixing said gaseous ozone during any one of said washing/rinsing steps of the appliance.

The present invention also relates to a method of use of said washing appliance, characterized in that said washing/rinsing steps of the appliance are controlled by said control system in order to carry out said mixing of gaseous ozone in said washing/rinsing liquid during one or more of the following washing/rinsing steps:
- ozone mixing is activated prior to any other sequence/step, if the water supplied does not have satisfactory microbiological characteristics, so as to abate the contaminant charge in the water;
- ozone mixing is activated during an initial liquid recirculation step, if the water supplied is pure, but said parts and components used for pharmaceutical production to be washed need to be decontaminated, with the possibility of eliminating a cleansing step and high temperatures of the washing liquid;
- ozone mixing is activated during a recirculation-only step following a normal washing/rinsing cycle in order to ensure complete disinfection of said parts and components for pharmaceutical production to be washed, while at the same time decontaminating the hydraulic circuit and the wash chamber;
- ozone mixing is activated during a first rinsing step following a washing step with detergents;
- ozone mixing is activated at the end of the washing steps and prior to draining the liquid, for the purpose of biologically treating the recirculation water;
- ozone mixing is activated during a self-cleaning cycle whenever one wants to restore conditions of perfect cleaning and disinfection of the hydraulic circuit and of the associated components.

It is a particular object of the present invention to provide a washing appliance adapted for application in pharmaceutical production and/or preclinical pharmaceutical research centres, for washing parts and components for pharmaceutical production, and a method of use of the appliance, as set out in the claims, which are an integral part of the present description.

### Brief description of the drawings

Further objects and advantages of the present invention will become apparent from the following detailed description of a preferred embodiment (and variants) thereof referring to the annexed drawings, which are only supplied by way of non-limiting example, wherein:
Figure 1 shows a functional equivalent diagram of the appliance of the present invention;
Figures 2 to 5 highlight views of one example of embodiment of the appliance.

In the drawings, the same reference numerals and letters identify the same items or components.

### Detailed description of some embodiments

The present invention relates to a washing appliance adapted for application in pharmaceutical production and/or preclinical pharmaceutical research centres, which can process components and bins and allows the use of the recirculation technique, while eliminating the problem of residual contaminants by adding to the process a decontamination step using ozone.

This is achieved through the application of a generator aboard the machine to produce gaseous ozone to be mixed with the recirculated water.

The technology for producing ozone and mixing it with water is *per se* known.

The sanitizing properties of ozone are also known and can be summarized as follows:
- Bacterial disinfection
- Viral inactivation
- Oxidation of dissolved minerals (iron, manganese, hydrogen sulphide...)
- Colour removal (oxidation)
- Odour removal (oxidation)
- Algae removal (oxidation)
- Oxidation of organic components (phenols, detergents, pesticides)
- Micro-flocculation of dissolved organic components (oxidation)
- Oxidation of inorganic components (cyanides, sulphites, nitrites)

It must also be pointed out that ozone production occurs simply through the use of a small quantity of filtered and dried compressed air (already available in the machine) and very little electric energy.

The following will describe the appliance as a whole and the specific application of ozone with reference to the annexed drawings.

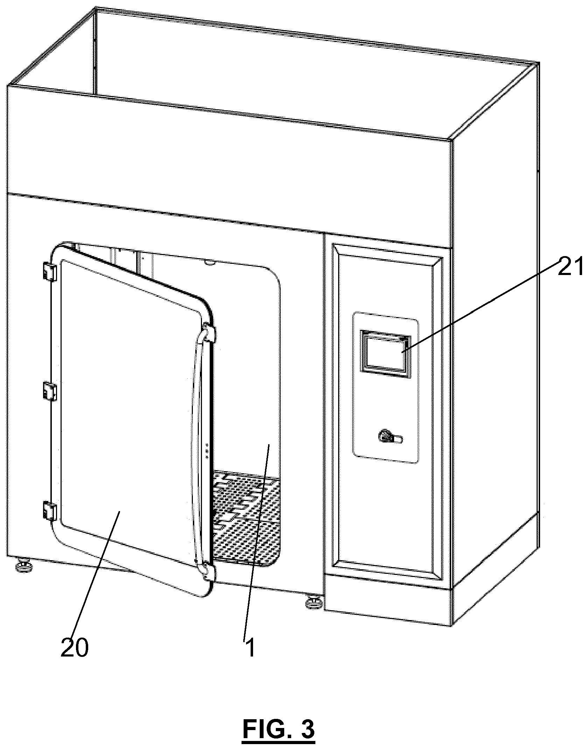

The appliance comprises a wash chamber 1 internally comprising an accumulation basin (tank) 2 at the bottom, underneath the base level. Inside the wash chamber 1, bins and carriages dedicated to the washing of components can be loaded through sealed door(s) 20 accessible on the side(s) of the machine (Fig. 3).

In the wash chamber 1 there are one or more of the following washing systems, provided with suitable holes or nozzles for delivering the washing liquid, which systems are connected to the hydraulic circuit of the appliance for circulating and recirculating the washing/rinsing liquid:
- general washing systems, in particular rotary rods 3 on the inner sidewalls of the chamber, adapted to spray washing liquids towards the inside of the chamber;
- washing systems dedicated to the washing of bins: in particular, an upper washing head 4 jutting out from the top wall of the chamber, and a lower washing head 5, jutting out from the bottom wall of the chamber;
- dedicated washing systems using dedicated carriages that can be inserted into the chamber, operating through a hydraulic connection of the manual or "quick-lock" type 6.

In one embodiment, the upper washing head 4, connected to a suitable liquid supply system 41, can move vertically within the wash chamber for the purpose of covering the entire load to be washed.

Preferably, in the chamber 1 there are a liquid level meter and a temperature probe (not shown in the drawings) co-operating with the electronic control system 18, which will be described below.

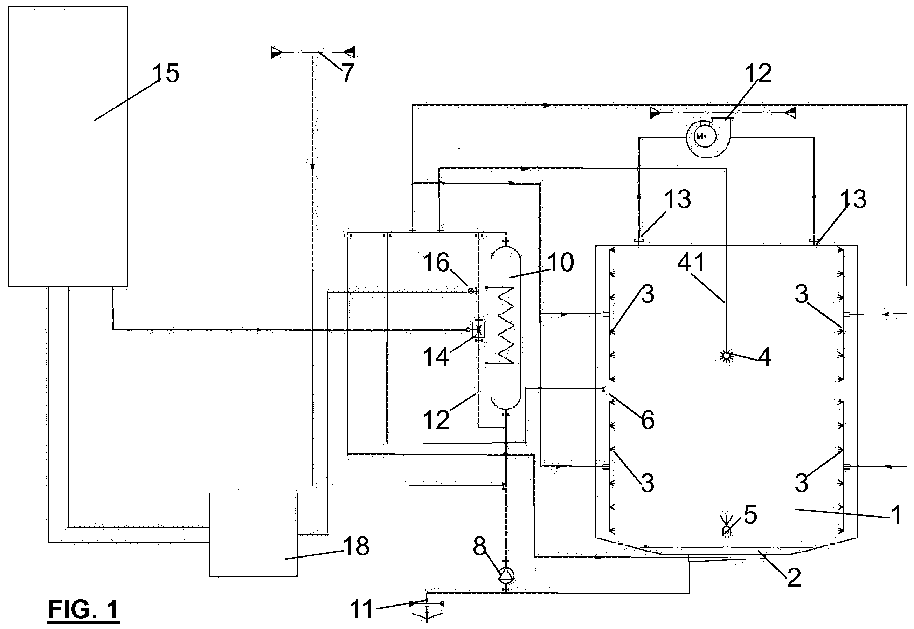

Figure 1 shows the pathlines of the hydraulic circuit.

The accumulation basin 2 is directly connected to a sanitary recirculation pump 8 capable of pressurizing the supplied water and feeding, whether alternately or simultaneously, one or more of the above-mentioned washing systems through the hydraulic recirculation circuit. The hydraulic circuit includes an in-line heat exchanger 10 for heating and maintaining the temperature of the washing/rinsing liquid received from the recirculation pump 8, a pressure switch and a temperature probe (not shown in the drawings). In the exchanger, heating is attained in any known manner.

The outlet of the heat exchanger 10 supplies the washing/rinsing liquid to the above-described washing systems of the chamber 1.

Through a drain valve 11 located at the lowest point of the hydraulic circuit, and connected to the accumulation basin 2, it is possible, by issuing an appropriate command, to completely drain the liquid circulating in the system.

On the top wall of the chamber there is an air extraction system 12 for extracting the air from inside the chamber by means of a fan intercepted by valves 13. This system ensures that the vapours generated during the process are extracted prior to opening the door(s) of the wash chamber.

There is at least one water supply point 7 for the hydraulic circuit, preferably connected to the delivery side of the recirculation pump 8.

Parallel to the heat exchanger 10, a bypass-line 12 of the hydraulic circuit is provided, which is equipped with a contact system connected to a gaseous ozone generation system.

The contact system includes mixing means, which in one embodiment consist of a known Venturi-effect mixer 14 appropriately sized to ensure proper mixing in water of the gaseous-phase ozone produced by the generator. Advantageously, the mixing system 14 is arranged along the exchanger by-pass line, but it may also be located elsewhere in the hydraulic circuit, provided that it allows mixing the ozone in the recirculated liquid.

The ozone generation system comprises a known gaseous-phase ozone generator 15, with a line for carrying the gaseous ozone towards the mixer 14. There is also an electronic control system 18 (e.g. a PLC) for controlling the ozone generator, and an ozone concentration meter 16 along the by-pass line 12, which provides the electronic control system 18 with a measurement of the ozone concentration in the liquid.

The electronic control system 18 also controls the recirculation pump 8 during the different steps of the washing cycle, changing the speed and pressure thereof accordingly, and the valves comprised in the hydraulic circuit for feeding the various spraying systems.

During any step and at any instant of the washing/rinsing cycle, it is possible, after having filled the water accumulation basin 2, to mix gaseous ozone with the water in the hydraulic system, according to the following sequence:
- The pump 8 turns on water recirculation and, under feedback control by the electronic control system 18, creates optimal mixing conditions in terms of pressure and flow rate.
- At this point, the electronic control system 18 interacts with the generator 15, which starts the production of gaseous ozone.
- The generator 15 turns on the supply of the produced ozone to the mixer 14, and the ozone begins mixing with the recirculated water.
- After a known and adjustable concentration of ozone in water is reached (which can be verified through the probe 16), the electronic control system 18 sends a signal to the generator 15 to stop ozone production by blocking the gas supply to the mixer.
- This step may be continued with the necessary times and combinations.
- When this step is the last one in the cycle sequence, a subsequent extraction step will be carried out in order to ensure complete extraction of any residue of gaseous ozone stagnating in the chamber, e.g. by means of the extraction system 12. In this case, it is preferable that the latter conveys the suction flow towards a dedicated duct under control of the final user, so as to avoid dispersing ozone directly into the environment.

The above-described operations are controlled through the electronic control system 18, the implementation of which is not a problem for a person skilled in the art on the basis of the information provided herein about the types of control to be carried out.

As aforesaid, the pressure of the washing liquid is adjustable according to the chosen cycle and the parts to be washed. In one exemplary embodiment, the adjustment range is 1 to 7 bar, since the bath recirculation pump is supplied by means of an inverter.

Ozone concentration in water may have values in the range of 1-2 ppm.

As far as cycle times are concerned, variability is high and may range from a few minutes to a few hours.

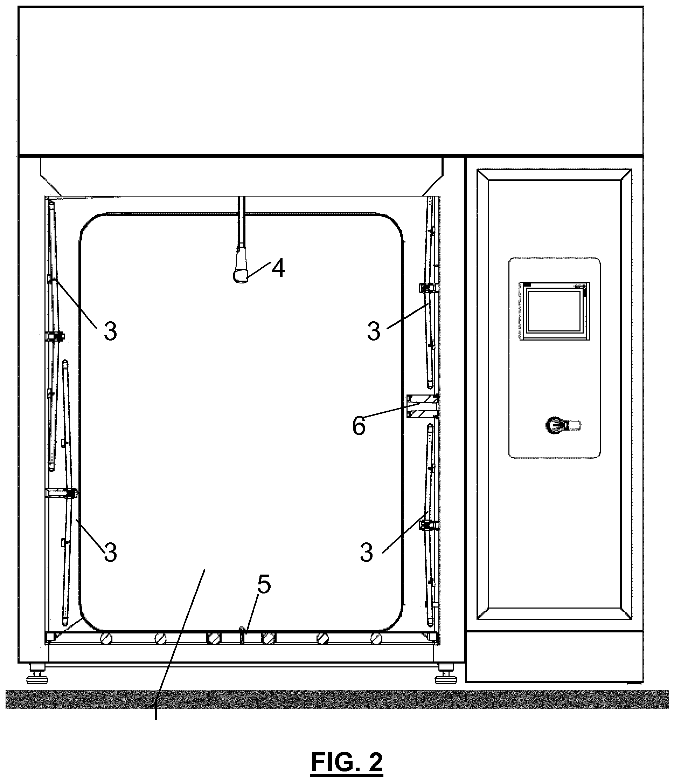

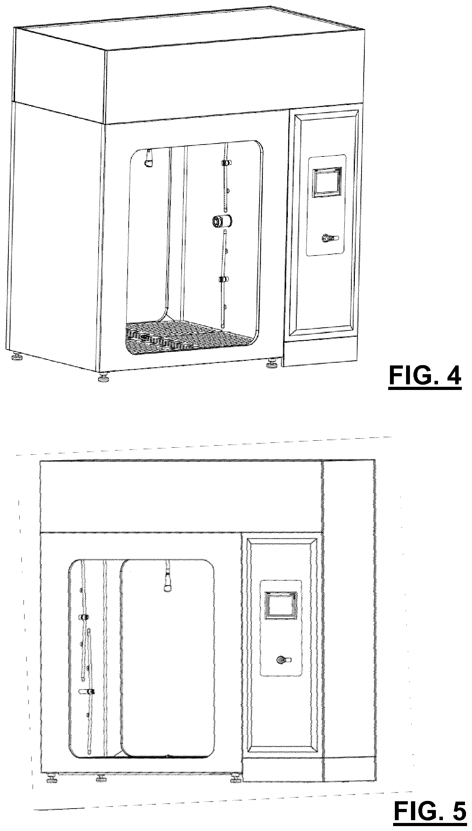

Figures 2-5 show some details of an exemplary embodiment of the washing appliance. It comprises the wash chamber 1, in which the various washing systems 3, 4, 5, 6 previously described are arranged. The chamber is accessible through a hermetically closed front door 20. The appliance is prearranged for receiving a second door on the opposite side. Around the wash chamber there are a number of technical compartments that can house the above-described other parts of the appliance. On the front wall there is a display 21, connected to the electronic control system 18, for controlling the various operations of the appliance.

As far as the operation of the appliance is concerned, the above-described machine can be used for many applications:
- The water available at the customer's site does not have satisfactory microbiological characteristics (presence of CFUs of contaminants): ozone mixing is activated prior to any other sequence/step, so as to abate the contaminant charge in the water. Therefore, during the initial recirculation a sufficient quantity of ozone is mixed, in an adjustable concentration, in order to decontaminate only the supplied amount of water, and then the normal washing step is carried out with the addition of detergent at an adequate temperature, if required to attain the desired washing and decontamination results on the load.
- The water available at the customer's site is pure, but the load needs to be decontaminated by using ozone only. In this case, ozone mixing is activated during the initial recirculation, until a sufficient and adjustable concentration value is obtained; only ozonated water is used for washing and decontaminating the load. In this case, it will be necessary to dose a larger quantity of ozone than at point 1), which will result in longer production times, but with the possibility of eliminating both the cleansing step and the need for high temperatures.
- After a normal washing/rinsing step (chemical aggression of dirt by detergent, and removal thereof by rinsing), a recirculation step with ozonated water is activated in order to ensure complete disinfection of the washed parts, simultaneously with recirculation in all circuits in order to decontaminate the circuits themselves and the wash chamber.
- Use of ozone after the detergent washing step: the oxidizing properties of ozone, which is particularly capable of oxidizing both organic and inorganic components, speed up the removal of any detergent traces from the components and from the system as a whole.
- At the end of the washing steps, prior to draining the liquid through the drain valve, ozone generation is activated in order to biologically treat the recirculation water.
- Self-cleaning cycle with only one ozone mixing step whenever one wants to restore conditions of perfect cleaning and disinfection of the wash chamber and of all circuits (including the heat exchanger).

The above-described solutions fulfil the need for solving the problems of the accumulation of contaminants and/or detergents in classic recirculation-type washing systems, in addition to offering the possibility to carry out cycles without using detergents and high temperatures.

The above-described non-limiting example of embodiment may be subject to variations without departing from the protection scope of the present invention, including all equivalent designs known to a man skilled in the art.

A possible embodiment variation is described here below.

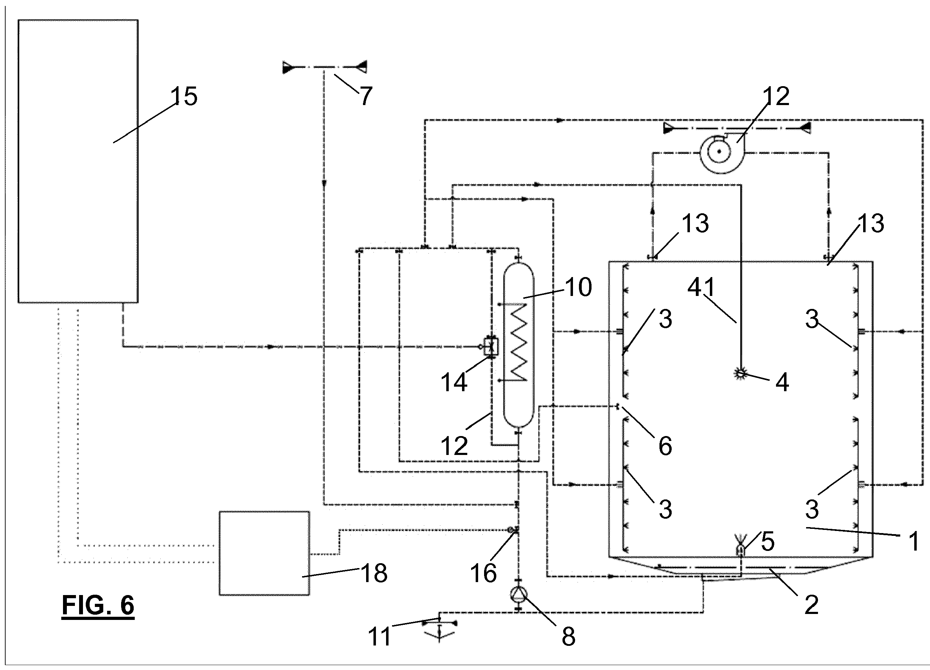

The control probe (16) of the ozone quantity can be placed at one point of the hydraulic circuit on the return of the washing liquid recirculation, for example upstream of the mixer 14 and of the heat exchanger 10, and downstream of the delivery of the recirculation pump 8 and of the outlet of the accumulation basin 2 (see figure 6), or between the outlet of the accumulation basin 2 and the pump 8.

During the production and mixing of Ozone the control probe (16), suitably placed on the return of the recirculation, verifies the increase of the residual mixed ozone and indirectly determines a control of the bacterial abatement that is being obtained in the plant.

Ozone is an extremely unstable molecule with a strong oxidizing power: this means that once produced it remains stable if and only if it does not find a bacterial charge or organic material to oxidize.

The increase in ozone concentration on the return of the recirculation (and therefore at the reading point of the probe 16) occurs only after the quantity of ozone produced and mixed with the washing liquid is greater than the part that is lost due to oxidation-reduction phenomena for combination with the bacterial load or organic material to be abated. Proceeding with the production and mixing and since the plant is isolated from the outside, the bacterial load and any organic charge present decrease, due to oxidation-reduction with ozone, until the microbiological conditions of the plant improve and the concentration of residual ozone on the return begins to rise. This means that if the mixed ozone value is read only downstream of the injection point 14 (see figure 1), the ozone may have already reached the recipe value, but there would be no indication regarding the amount of residual ozone after recirculation and possible oxidation-reduction with the present contaminants. With this type of control, on the other hand, the time required to reach the set ozone concentration is therefore not only linked to the production and mixing operations between the generation plant and the machine, but also linked to the level of charge abatement of contaminant present in the chamber. Depending on the starting level of contamination in the plant, the control system 18 is able to determine the time of achievement of the set value, to guarantee improved microbiological conditions of the plant (qualitatively and not quantitatively).

Once this value is reached, it is possible to set a maintenance phase according to the needs and continue with the necessary combinations, keeping the ozone level monitored and modulated.

As mentioned above, the control system is therefore also able to provide an indication of the level of bacterial or contaminant charge present in the washing chamber, based on the measurement of the time required to reach the desired ozone concentration level in the washing liquid, starting from the beginning of delivery.

Therefore it is particularly advantageous to use the washing apparatus object of the invention in the context of the variant described above, being able to better control the ozone delivery time and the amount of ozone injected, increasing the effectiveness of the washing method, in any of the washing / rinsing phases described above. The elements and features shown in the various preferred embodiments may be combined together without however departing from the protection scope of the present invention.

From the above description, those skilled in the art will be able to produce the object of the invention without introducing any further construction details.

## Claims

1. Washing appliance adapted for application in pharmaceutical production and/or preclinical pharmaceutical research centres, and adapted for the execution of washing/rinsing steps on parts and components for pharmaceutical production, comprising a hydraulic circuit for the circulation of washing/rinsing liquid and a recirculation pump (8) in the hydraulic circuit, **characterized in that** it comprises:
- a wash chamber (1) adapted to contain said parts and components for pharmaceutical production to be washed, said chamber comprising a hermetically closed access door (20);
- one or more washing systems, internal to the wash chamber and connected to said hydraulic circuit, and adapted to supply washing liquid into the chamber;
- an accumulation basin (2) at the bottom of the wash chamber, for accumulating the washing/rinsing liquid, connected to said recirculation pump (8);
- a heat exchanger (10) for heating or maintaining the temperature of the washing/rinsing liquid received through said recirculation pump (8) and supplied to said one or more washing systems;
- a generation system (15) adapted to generate gaseous ozone;
- mixing means (14) adapted to mix said gaseous ozone with the washing/rinsing liquid and arranged in said hydraulic circuit;
- a control system (18) adapted to control said washing/rinsing steps of the appliance, and adapted to control said gaseous ozone generation system (15) for mixing said gaseous ozone during any one of said washing/rinsing steps of the appliance.

2. Washing appliance according to claim 1, wherein said mixing means comprise a Venturi-effect mixer (14) arranged along a by-pass line (12) of the hydraulic circuit, the by-pass line by-passing said heat exchanger (10).

3. Washing appliance according to claim 1, wherein said one or more washing systems comprise:
- one or more rotary rods (3) on the internal sidewalls of the chamber; and/or
- an upper washing head (4) jutting out from the top wall of the chamber; and/or
- a lower washing head (5) jutting out from the bottom wall of the chamber; and/or
- dedicated washing systems using dedicated carriages that can be inserted into the chamber through a manual or "quick-lock" hydraulic connection (6).

4. Washing appliance according to claim 1, comprising a drain valve (11) located at the lowest point of the hydraulic circuit, connected to the accumulation basin (2), adapted to drain the liquid circulating in the hydraulic circuit.

5. Washing appliance according to claim 1, comprising an air extraction system (12) for extracting the air from inside the chamber, arranged on the top wall of the chamber.

6. Washing appliance according to claim 1, comprising at least one water supply point (7) for the hydraulic circuit.

7. Washing appliance according to claim 1, comprising means for measuring the ozone concentration (16) of the hydraulic circuit in the by-pass line (12), adapted to provide said control system (18) with a measurement of the ozone concentration in the water.

8. Method of use of a washing appliance according to any one of the preceding claims, **characterized in that** said washing/rinsing steps of the appliance are controlled by said control system (18) so as to carry out said mixing of gaseous ozone in said washing/rinsing liquid during one or more of the following washing/rinsing steps:
- ozone mixing is activated prior to any other sequence/step, if the water supplied does not have satisfactory microbiological characteristics, so as to abate the contaminant charge in the water;
- ozone mixing is activated during an initial liquid recirculation step, if the water supplied is pure, but said parts and components for pharmaceutical production to be washed need to be decontaminated, with the possibility of eliminating a cleansing step and high temperatures of the washing liquid;
- ozone mixing is activated during a recirculation-only step following a normal washing/rinsing cycle in order to ensure complete disinfection of said parts and components for pharmaceutical production to be washed, while at the same time decontaminating the hydraulic circuit and the wash chamber;
- ozone mixing is activated during a first rinsing step following a washing step with detergents;
- ozone mixing is activated at the end of the washing steps and prior to draining the liquid, for the purpose of biologically treating the recirculation water;
- ozone mixing is activated during a self-cleaning cycle whenever one wants to restore conditions of perfect cleaning and disinfection of the hydraulic circuit and of the associated components.
